# EUROPÄISCHE PATENTSCHRIFT

(11) **EP 1 006 992 B1**
(45) Veröffentlichungstag und Bekanntmachung des Hinweises auf die Patenterteilung: **02.05.2002**
(21) Anmeldenummer: 98946359.1
(22) Anmeldetag: 17.08.1998
(51) Int. Cl.: A61K 7/16, A61K 7/50

(54) **VERWENDUNG VON FETTSÄUREPOLYGLYCOLESTERSULFATEN**
USE OF FATTY ACID POLYGLYCOL ESTER SULPHATES
UTILISATION DE SULFATES D'ESTER POLYGLYCOL D'ACIDES GRAS

(30) Priorität: 25.08.1997 DE 19736906; 25.09.1997 DE 19741911; 23.10.1997 DE 19746779
(43) Veröffentlichungstag der Anmeldung: 14.06.2000
(73) Patentinhaber: Cognis Deutschland GmbH & Co. KG, 40589 Düsseldorf (DE)
(72) Erfinder: ANSMANN, Achim, D-40699 Erkrath (DE); HENSEN, Hermann, D-42781 Haan (DE)
(86) Internationale Anmeldenummer: EP9805213
(87) Internationale Veröffentlichungsnummer: WO9909942

(56) Entgegenhaltungen:
- WO-A-93/06081
- WO-A-95/23582

## Beschreibung

### Gebiet der Erfindung

Die Erfindung betrifft den Einsatz von Fettsäurepolyglycolestersulfaten zur Herstellung von Mund- und Zahnpfiegemitteln sowie Mittel, die die Fettsäurepolyglycolestersulfate als schäumende Tensidkomponente enthalten.

### Stand der Technik

Mund- und Zahnpflegemittel, wie beispielsweise Zahnpasten oder die in letzter Zeit zunehmend im Markt befindlichen Zahnputzgele, enthalten üblicherweise als mengenmäßige Hauptbestandteile Schleif- und Poliermittel zur mechanischen Entfernung von Verunreinigungen und Plaque (z.B. Kreide), Feuchthaltemittel (z.B. Glycerin) und Tenside, wie beispielsweise Laurylsulfat, Laurylethersulfat oder Alkylpolyglucoside. Die Aufgabe dieser letzten Komponente besteht in erster Linie darin, einen kräftigen Reibschaum zu entwickeln. Bei der Auswahl der Tenside kommen also in erster Linie schaumstarke Produkte in Frage, die zudem toxikologisch unbedenklich und ausgesprochen mundschleimhautverträglich sein müssen. Ein weiteres Problem besteht ferner darin, daß nur solche Tenside eingesetzt werden können, die geschmacksneutral sind bzw, deren Eigengeschmack durch Aromastoffe problemlos überdeckt werden kann. Im Markt besteht daher ein besonderes Interesse an Tensiden, die das gewünschte Anforderungsprofil erfüllen und zu vergleichbaren Ergebnissen bei verminderter Einsatzkonzentration führen. Die Aufgabe der Erfindung hat darin bestanden, hierfür eine Lösung bereitzustellen.

### Beschreibung der Erfindung

Gegenstand der Erfindung ist die Verwendung von Fettsäurepolyglycolestersulfaten der Formel **(I)**,

**R**^{**1**}**CO(AO)**_{**x**}**SO**_{**3**}**X (I)**

in der R¹CO für einen linearen oder verzweigten, gesättigten oder ungesättigten Acylrest mit 6 bis 22 Kohlenstoffatomen, x für Zahlen von durchschnittlich 1 bis 3 und AO für einen CH₂CH₂O-, CH₂CH(CH₃)O- und/oder CH(CH₃)CH₂O-Rest und X für ein Alkali- und/oder Erdalkalimetall, Ammonium, Alkylammonium, Alkanolammonium oder Glucammonium steht, zur Herstellung von Mund- und Zahnpfiegemitteln, speziell Zahnpasten und Zahnputzgele.

Überraschenderweise wurde gefunden, daß Fettsäurepolyglycolestersulfate nicht nur eine besonders vorteilhafte Schleimhautverträglichkeit aufweisen, sondern zudem einen besonders stabilen und cremigen Schaum entwickeln und eine zufriedenstellende geschmackliche Qualität aufweisen. In Abmischung mit anderen Tensiden, insbesondere Alkylethersulfaten, Monoglyceridethersulfaten und/oder Alkyloligoglucosiden wird eine synergistische Verstärkung der genannten Eigenschaften beobachtet. Hierdurch lassen sich Formulierungen herstellen, die bei gleichem Leistungsvermögen einen verminderten Tensidanteil aufweisen.

### Fettsäurepolyglycolestersulfate

Fettsäurepolyglycolestersulfate werden durch Sulfatierung der entsprechenden Fettsäurepolyglycolester hergestellt. Diese wiederum sind nach den einschlägigen präparativen Verfahren der organischen Chemie erhältlich. Hierzu wird Ethylenoxid, Propylenoxid oder deren Gemisch - in random- oder Blockverteilung - an die entsprechenden Fettsäuren angelagert, wobei diese Reaktion säurekatalysiert, vorzugsweise aber in Gegenwart von Basen, wie z.B. Natriummethylat oder calciniertem Hydrotalcit erfolgt. Wird ein Alkoxylierungsgrad von 1 gewünscht, können die Zwischenprodukte auch durch Veresterung der Fettsäuren mit einem entsprechenden Alkylenglycol hergestellt werden. Die Sulfatierung der Fettsäurepolyglycolester kann in an sich bekannter Weise mit Chlorsulfonsäure oder vorzugsweise gasförmigem Schwefeltrioxid durchgeführt werden, wobei das molare Einsatzverhältnis zwischen Fettsäurepolyglycolester und Sulfatierungsmittel im Bereich von 1 : 0,95 bis 1:1,2, vorzugsweise 1 : 1 bis 1 : 1,1 und die Reaktionstemperatur 30 bis 80 und vorzugsweise 50 bis 60°C betragen kann. Es ist ferner möglich, die Fettsäurepolyglycolester zu untersulfatieren, d.h. deutlich weniger Sulfatierungsmittel einzusetzen, als dies für eine vollständige Umsetzung stöchiometrisch erforderlich wäre. Wählt man beispielsweise molare Einsatzmengen von Fettsäurepolyglycolester zu Sulfatierungsmittel von 1 : 0,5 bis 1 : 0,95 werden Mischungen von Fettsäurepolyglycolestersulfaten und Fettsäurepolyglycolestem erhalten, die für eine ganze Reihe von Anwendungen ebenfalls vorteilhaft sind. Um eine Hydrolyse zu vermeiden ist es dabei sehr wichtig, die Neutralisation bei einem pH-Wert im Bereich von 5 bis 9, vorzugsweise 7 bis 8 durchzuführen. Typische Beispiele für geeignete Ausgangsstoffe sind die Anlagerungsprodukte von 1 bis 3 Mol Ethylenoxid und/oder Propylenoxid, vorzugsweise aber die Addukte mit 1 Mol Ethylenoxid oder 1 Mol Propylenoxid an Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die dann wie oben beschrieben sulfatiert und neutralisiert werden. Vorzugsweise werden Fettsäurepolyglycolestersulfate der Formel (I) eingesetzt, in der R¹CO für einen Acylrest mit 12 bis 18 Kohlenstoffatomen, x für durchschnittlich 1 oder 2, AO für eine CH₂CH₂O-Gruppe und X für Natrium oder Ammonium steht, wie beispielsweise Laurinsäure+1EO-sulfat-Natriumsalz, Laurinsäure+1EOsulfat-Ammoniumsalz, Kokosfettsäure+1EO-sulfat-Natriumsalz, Kokosfettsäure+1EO-sulfat-Ammoniumsalz, Talgfettsäure+1EO-sulfat-Natriumsalz, Talgfettsäure+1EO-sulfat-Ammoniumsalz sowie deren Mischungen.

### Tenside

Die Fettsäurepolyglycolestersulfate können alleine eingesetzt werden, ein besonderer Vorteil ergibt sich jedoch durch die synergistische Schaumverstärkung in Abmischung mit einem breiten Spektrum anderer anionischer, nichtionischer, amphoterer und/oder zwitterionischer Tenside. Typische Beispiele für anionische Tenside sind Seifen, Alkylbenzolsulfonate, Alkansulfonate, Olefinsulfonate, Alkylethersulfonate, Glycerinethersulfonate; α-Methylestersulfonate, Sulfofettsäuren, Glycerinethersulfate, Hydroxymischethersulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofem die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **amphotere bzw. zwiltterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen.

### Alkyl(ether)sulfate

In einer bevorzugten Ausführungsform der Erfindung werden die Fettsäurepolyglycolestersulfate zusammen mit Alkylsulfaten bzw. Alkylethersulfaten eingesetzt. Diese stellen bekannte anionische Tenside dar, die großtechnisch durch SO₃- oder Chlorsulfonsäure (CSA)-Sulfatierung von Fettalkoholen bzw. Fettalkohol- oder Oxoalkoholpolyglycolethem und nachfolgende Neutralisation hergestellt werden. Im Sinne der Erfindung kommen Alkylsulfate bzw. Ethersulfate in Betracht, die der Formel **(II)** folgen,

**R**^{**2**}**O-(CH**_{**2**}**CH**_{**2**}**O)**_{**n**}**SO**_{**3**}**X (II)**

in der R¹ für einen linearen oder verzweigten Alkyl- und/oder Alkenylrest mit 6 bis 22 Kohlenstoffatomen, n für 0 oder Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Alkylsulfaten wie beispielsweise Laurylsulfat oder Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

### Monoglycerid(ether)sulfate

In einer weiteren bevorzugten Ausführungsform der Erfindung werden die Fettsäurepolyglycolestersulfate zusammen mit Monoglyceridsulfaten bzw. Monoglyceridethersulfaten eingesetzt. Diese stellen ebenfalls bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **EP-B1 0561825, EP-B1 0561999** (Henkel)]. Die neutralisierten Stoffe können - falls gewünscht - einer Ultrafiltration unterworfen werden, um den Elektrolytgehalt auf ein gewünschtes Maß zu vermindern **[DE-A1 42 04 700** (Henkel)]. Übersichten zur Chemie der Monoglyceridsulfate sind beispielsweise von A.K.Biswas et al. in **J.Am.Oil.Chem.Soc. 37, 171 (1960)** und F.U.Ahmed **J.Am.Oil.Chem.Soc. 67,** **8 (1990)** erschienen. Die im Sinne der Erfindung einzusetzenden Monoglycerid(ether)sulfate folgen der Formel (III) 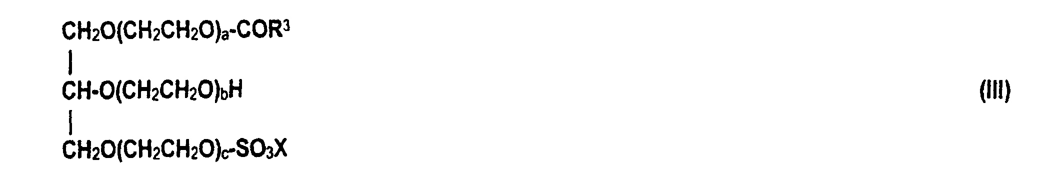 in der R³CO für einen linearen oder verzweigten Acylrest mit 6 bis 22 Kohlenstoffatomen, a, b und c in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel **(III)** eingesetzt, in der R³CO für einen linearen Acylrest mit 8 bis 18 Kohlenstoffatomen steht.

### Alkyl- und/oder Alkenyloligoglykoside

In einer dritten bevorzugten Ausführungsform der Erfindung werden die Fettsäurepolyglycolestersulfate zusammen mit Alkyl- und/oder Alkenyloligoglykosiden eingesetzt. Diese stellen bekannte nichtionische Tenside dar, die der Formel **(IV)** folgen,

**R**^{**4**}**O-[G]**_{**p**} **(IV)**

in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(IV)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁴ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Im Sinne der Erfindung können die Fettsäurepolyglycolestersulfate alleine oder in Abmischung mit den genannten Co-Tensiden in Mengen von 1 bis 10, vorzugsweise 2 bis 5 Gew.-% - bezogen auf die Zubereitungen und berechnet als Feststoff - eingesetzt werden. Das Einsatzverhältnis der Fettsäurepolyglycolestersulfate und der Co-Tenside untereinander kann 10 : 90 bis 90 : 10, vorzugsweise 25 : 75 bis 75 : 25 und insbesondere 40 : 60 bis 60 : 40 Gewichtsteile betragen.

### Schleif- und Poliermittel

Als Schleif- und Poliermittel können die erfindungsgemäßen Mittel Kreide, Dicalciumphosphat, Natriumbicarbonat, unlösliches Natriummetaphosphat, Aluminiumsilicat, Schichtsilicate, Hydrotalcite, Calciumpyrophosphat, feinteilige Kunstharze, Kieselsäuren, Aluminiumoxid, Aluminiumoxidtrihydrat, Talkum, Zeolithe, Magnesiumaluminiumsilicat (Veegum®), Calciumsulfat, Magnesiumcarbonat und/oder Magnesiumoxid enthalten. Der Anteil der Schleif- und Poliermittel kann 5 bis 30, vorzugsweise 15 bis 25 Gew.-% - bezogen auf die fertigen Zubereitungen - betragen.

### Aromakomponenten

Neben den genannten Tensiden kommen als weitere Hilfs- und Zusatzstoffe auch Aromakomponenten in Frage, beispielsweise Pfefferminzöl, Krauseminzöl, Anisöl, Sternanisöl, Kümmelöl, Eukalyptusöl, Fenchel, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle, wie z.B. Menthol, Carvon, Anethol, Cineol, Eugenol, Zimtaldehyd, Cargophyllen, Geraniol, Citronellol, Linalool, Salven, Thymol, Terpinan, Terpinol, Methylchavicol und Methylsalicylat. Weitere geeignete Aromen sind z.B. Methylacetat, Vanillin, lonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam. Der Anteil der Aromastoffe kann 0,1 bis 3, vorzugsweise 0,2 bis 1 Gew.-% - bezogen auf die fertigen Zubereitungen - betragen.

### Weitere Inhaltsstoffe

Ferner kommen für den Einsatz insbesondere in Zahnpasten als Hilfs- und Zusatzstoffe Feuchthaltemittel wie z.B. Sorbit, Glycerin oder Polyethylenglycole, Konsistenzregler, desodorierende Wirkstoffe, Quellstoffe und Bindemittel, wie z.B. Carboxymethylcellulose oder Xanthan Gum, Wirkstoffe gegen Mund- und Zahnerkrankungen, wasserlösliche Fluorverbindungen wie z.B. Natriumfluorid oder Natriummonofluorphosphat in Betracht. Der Anteil der Hilfs- und Zusatzstoffe ist an sich unkritisch und richtet sich nach der Art des schließlich zu konfektionierenden Mittels. Üblicherweise wird der Anteil 5 bis 98 und vorzugsweise 80 bis 90 Gew.-% - bezogen auf die fertigen Zubereitungen - betragen.

Eine typische Zahnpaste weist im Sinne eines weiteren Gegenstandes der Erfindung folgende typische Zusammensetzung auf (Wasser ad 100 Gew.-%):
15 bis 25 Gew.-% Schleif- und Poliermittel,
30 bis 65 Gew.-% Feuchthaltemittel,
1 bis 10 Gew.-% Fettsäurepolyglycolestersulfate, ggf. in Abmischungen mit Co-Tensiden,
1 bis 2 Gew.-% Aromastoffe und
0 bis 5 Gew.-% weitere Hilfsstoffe,
mit der Maßgabe, daß sich die Einsatzmengen zu 100 Gew.-% ergänzen.

### Beispiele

Die erfindungsgemäße Verwendung wird an Hand der nachfolgenden drei Rezepturbeispiele illustriert.

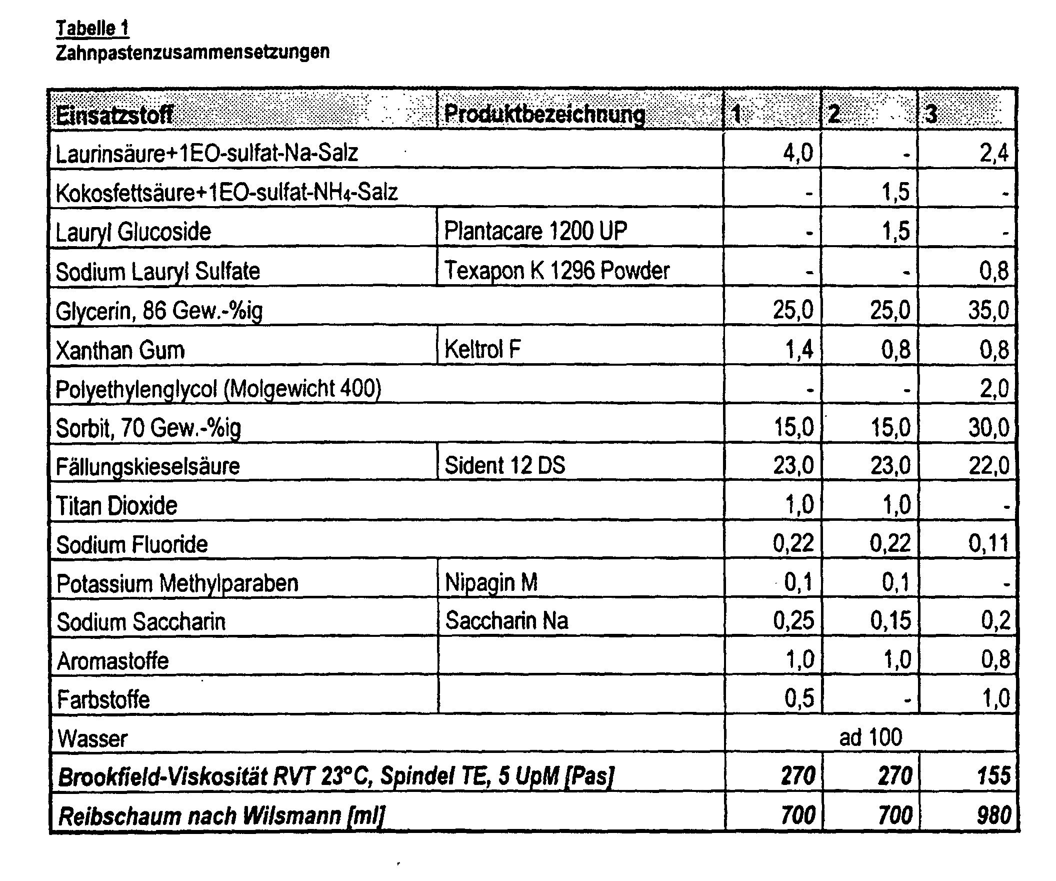

## Patentansprüche

1. Verwendung von Fettsäurepolyglycolestersulfaten der Formel **(I)**,
**R**^{**1**}**CO(AO)**_{**x**}**SO**_{**3**}**X (I)**
in der R¹CO für einen linearen oder verzweigten, gesättigten oder ungesättigten Acylrest mit 6 bis 22 Kohlenstoffatomen, x für Zahlen von durchschnittlich 1 bis 3 und AO für einen CH₂CH₂O-, CH₂CH(CH₃)O- und/oder CH(CH₃)CH₂O-Rest und X für ein Alkali- und/oder Erdalkalimetall, Ammonium, Alkylammonium, Alkanolammonium oder Glucammonium steht, zur Herstellung von Mund- und Zahnpflegemitteln.

2. Verwendung nach Anspruch 1, **dadurch gekennzeichnet, daß** man Fettsäurepolyglycolestersulfate der Formel (I) einsetzt, in der R¹CO für einen Acylrest mit 12 bis 18 Kohlenstoffatomen, x für durchschnittlich 1 oder 2, AO für eine CH₂CH₂O-Gruppe und X für Natrium oder Ammonium steht.

3. Verwendung nach den Ansprüchen 1 und 2, **dadurch gekennzeichnet, daß** man zusätzlich Alkyl-(ether)sulfate der Formel (II) einsetzt,
**R**^{**2**}**O-(CH**_{**2**}**CH**_{**2**}**O)**_{**n**}**SO**_{**3**}**X (II)**
in der R² für einen linearen oder verzweigten Alkyl- und/oder Alkenylrest mit 6 bis 22 Kohlenstoffatomen, n für 0 oder Zahlen von 1 bis 10 und X für ein Alkali- und/oder Erdalkalimetall, Ammonium, Alkylammonium, Alkanolammonium oder Glucammonium steht.

4. Verwendung nach den Ansprüchen 1 bis 3, **dadurch gekennzeichnet, daß** man zusätzlich Monoglycerid(ether)sulfate der Formel (III) einsetzt, 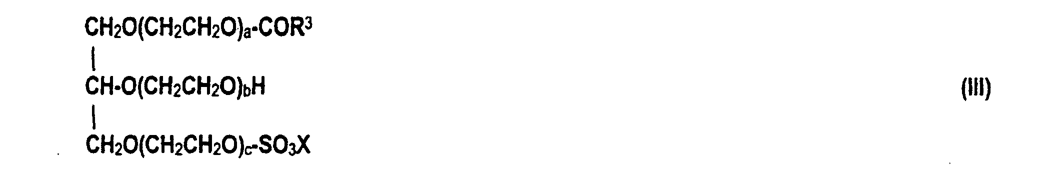 in der R³CO für einen linearen oder verzweigten Acylrest mit 6 bis 22 Kohlenstoffatomen, a, b und c in Summe für 0 oder für Zahlen von 1 bis 30 und X für ein Alkali- oder Erdalkalimetall steht.

5. Verwendung nach den Ansprüchen 1 bis 4, **dadurch gekennzeichnet, daß** man zusätzlich Alkylund Alkenyloligoglykoside der Formel (IV) einsetzt,
**R**^{**4**}**O-[G]**_{**p**} **(IV)**
in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

6. Verwendung nach den Ansprüchen 1 bis 5, **dadurch gekennzeichnet, daß** man die Fettsäurepolyglycolestersulfate alleine oder in Abmischung mit den Co-Tensiden in Mengen von 1 bis 10 Gew.-% - bezogen auf die fertigen Zubereitungen - einsetzt.

7. Verwendung nach den Ansprüchen 1 bis 6, **dadurch gekennzeichnet, daß** man Fettsäurepolyglycolestersulfate und die Co-Tenside im Gewichtsverhältnis 10 : 90 bis 90 : 10 einsetzt.

8. Verwendung nach den Ansprüchen 1 bis 7, **dadurch gekennzeichnet, daß** man zusätzlich Schleif- und Poliermittel einsetzt, die ausgewählt sind aus der Gruppe, die gebildet wird von Kreide, Dicalciumphosphat, Natriumbicarbonat, unlöslichem Natriummetaphosphat, Aluminiumsilicat, Schichtsilicaten, Hydrotalciten, Calciumpyrophosphat, feinteiligen Kunstharzen, Kieselsäuren, Aluminiumoxid, Aluminiumoxidtrihydrat, Talkum, Zeolithen, Magnesiumaluminiumsilicat, Calciumsulfat, Magnesiumcarbonat und/oder Magnesiumoxid.

9. Verwendung nach den Ansprüchen 1 bis 8, **dadurch gekennzeichnet, daß** man zusätzlich Glycerin, Sorbit und/oder Polyethylenglycole als Feuchthaltemittel einsetzt.

10. Mund- und Zahnpflegemittel, enthaltend
15 bis 25 Gew.-% Schleif- und Poliermittel,
30 bis 65 Gew.-% Feuchthaltemittel,
1 bis 10 Gew.-% Fettsäurepolyglycolestersulfate, ggf. in Abmischungen mit Co-Tensiden,
1 bis 2 Gew.-% Aromastoffe und
0 bis 5 Gew.-% weitere Hilfsstoffe,
mit der Maßgabe, daß sich die Einsatzmengen zu 100 Gew.-% ergänzen.

## Claims

1. The use of fatty acid polyglycol ester sulfates corresponding to formula (I):
R¹CO(AO)ₓSO₃X (I)
in which R¹CO is a linear or branched, saturated or unsaturated acyl group containing 6 to 22 carbon atoms, x has an average value of 1 to 3 and AO is a CH₂CH₂O-, CH₂CH(CH₃)O- and/or CH(CH₃)CH₂O group and X is an alkali and/or alkaline earth metal, ammonium, alkylammonium, alkanolammonium or glucammonium,
for the production of oral and dental care compositions, especially tooth pastes and tooth cleaning gels.

2. The use claimed in claim 1, **characterized in that** fatty acid polyglycol ester sulfates corresponding to formula (I), in which R¹CO is an acyl group containing 12 to 18 carbon atoms, x has an average value of 1 or 2, AO is a CH₂CH₂O group and X is sodium or ammonium, are used.

3. The use claimed in claims 1 and 2, **characterized in that** alkyl (ether) sulfates corresponding to formula (II):
R²O-(CH₂CH₂O)ₙSO₃X (II)
in which R² is a linear or branched alkyl and/or alkenyl group containing 6 to 22 carbon atoms, n = 0 or a number of 1 to 10 and X is an alkali metal and/or alkaline earth metal, ammonium, alkylammonium, alkanolammonium or glucammonium,
are aditionally used.

4. The use claimed in claims 1 to 3, **characterized in that** monoglyceride (ether) sulfates corresponding to formula (III): 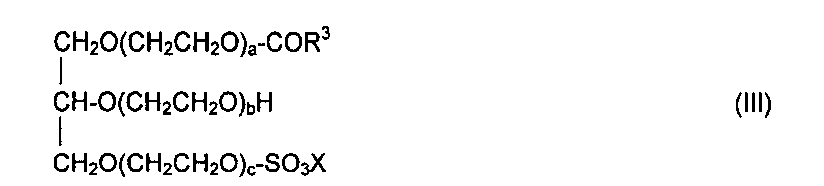 in which R³CO is a linear or branched acyl group containing 6 to 22 carbon atoms, a, b and c together stand for 0 or for numbers of 1 to 30 and X is an alkali metal or alkaline earth metal,
are additionally used.

5. The use claimed in claims 1 to 4, **characterized in that** alkyl and alkenyl oligoglycosides corresponding to formula (IV):
R⁴O-[G]ₚ (IV)
in which R⁴ is an alkyl and/or alkenyl group containing 4 to 22 carbon atoms, G is a sugar unit containing 5 or 6 carbon atoms and p is a number of 1 to 10,
are additionally used.

6. The use claimed in claims 1 to 5, **characterized in that** the fatty acid polyglycol ester sulfates are used on their own or in combination with co-surfactants in quantities of 1 to 10% by weight, based on the final compositions.

7. The use claimed in claims 1 to 6, **characterized in that** the fatty acid polyglycol ester sulfates and the co-surfactants are used in a ratio by weight of 10:90 to 90:10.

8. The use claimed in claims 1 to 7, **characterized in that** abrasives and polishes selected from the group consisting of chalk, dicalcium phosphate, sodium bicarbonate, insoluble sodium metaphosphate, aluminium silicate, layer silicates, hydrotalcites, calcium pyrophosphate, fine-particle synthetic resins, silicas, aluminium oxide, aluminium oxide trihydrate, talcum, zeolites, magnesium aluminium silicate, calcium sulfate, magnesium carbonate and/or magnesium oxide are additionally used.

9. The use claimed in claims 1 to 8, **characterized in that** glycerol, sorbitol and/or polyethylene glycols are additionally used as humectants.

10. Oral and dental care compositions containing
15 to 25% by weight abrasives and polishes,
30 to 65% by weight humectants,
1 to 10% by weight fatty acid polyglycol ester sulfates, optionally in combination with co-surfactants,
1 to 2% by weight flavouring agents and
0 to 5% by weight other auxiliaries,
with the proviso that the quantities used add up to 100% by weight.

## Revendications

1. Utilisation de sulfates d'esters de polyglycol et d'acide gras de formule (I)
R¹CO(AO)ₓSO₃X (I)
dans laquelle R¹CO représente un reste acyle linéaire ou ramifié, saturé ou non saturé, ayant de 6 à 22 atomes de carbone, x représente des nombres de, en moyenne, 1 à 3 et AO représente un reste CH₂CH₂O-, CH₂CH(CH₃)O- et/ou CH(CH₃)CH₂O- et X représente un métal alcalin et/ou alcalino-terreux, un ammonium, un alkylammonium, un alkanolammonium ou un glucammonium,
en vue de la préparation de produits de soins buccaux et dentaires.

2. Utilisation selon la revendication 1,
**caractérisée en ce qu'**
on met en oeuvre des sulfates d'esters de polyglycol et d'acide gras de formule (I) dans laquelle R¹CO représente un reste acyle ayant de 12 à 18 atomes de carbone, x représente des nombres de, en moyenne, 1 ou 2, AO représente un groupe CH₂CH₂O-, et X représente le sodium ou l'ammonium.

3. Utilisation selon l'une quelconque des revendications 1 et 2,
**caractérisée en ce qu'**
on met en oeuvre en supplément des sulfates (d'éther) d'alkyle de formule (II),
R²O-(CH₂CH₂O)ₙSO₃X (II)
dans laquelle R² représente un reste alkyle et/ou alkényle linéaire ou ramifié, ayant de 6 à 22 atomes de carbone, n représente O ou des nombres allant de 1 à 10 et X représente un métal alcalin et/ou alcalino-terreux, l'ammonium, l'alkylammonium, un alkanolammonium, ou un glucammonium.

4. Utilisation selon l'une quelconque des revendications 1 à 3,
**caractérisée en ce qu'**
on met en oeuvre en supplément des sulfates (d'éther) de monoglycéride de formule (III), 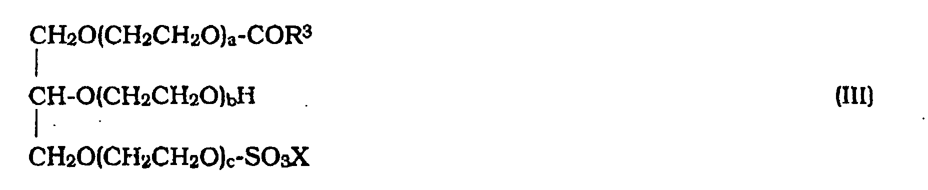 dans laquelle R³CO représente un reste acyle linéaire ou ramifié ayant de 6 à 22 atomes de carbone, a, b et c représentent globalement 0 ou des nombres allant de 1 à 30, de préférence de 2 à 10 et X représente un métal alcalin ou alcalino-terreux.

5. Utilisation selon l'une quelconque des revendications 1 à 4,
**caractérisée en ce qu'**
on met en oeuvre en supplément des alkyl- et alkényloligoglycosides de formule (IV).
R⁴O-[G]ₚ (IV)
dans laquelle R⁴ représente un reste alkyle et/ou alkényle ayant de 4 à 22 atomes de carbone, G représente un reste de sucre ayant 5 ou 6 atomes de carbone, et p représente des nombres allant de 1 à 10.

6. Utilisation selon l'une quelconque des revendications 1 à 5,
**caractérisée en ce qu'**
on met en oeuvre les sulfates d'ester de polyglycol et d'acide gras tout seuls ou en mélange avec les co-agents tensioactifs en quantités allant de 1 à 10 % en poids par rapport aux préparations terminées.

7. Utilisation selon l'une quelconque des revendications 1 à 6,
**caractérisée en ce qu'**
on met en oeuvre des sulfates d'ester de polyglycol et d'acide gras et les co-agents tensioactifs dans un rapport pondéral allant de 10:90 à 90:10.

8. Utilisation selon l'une quelconque des revendications 1 à 7,
**caractérisée en ce qu'**
on met en oeuvre en supplément des produits abrasifs et de polissage qui sont choisis dans le groupe qui est formé par la craie, le phosphate dicalcique, le bicarbonate de sodium, le métaphosphate de sodium in-soluble. le silicate d'aluminium, les silicates lamellaires, les hydratalcites, le pyrophosphate de calcium, les résines artificielles finement divisées, les acides siliciques, l'oxyde d'aluminium, le trihydrate d'oxyde d'aluminium, le talc, les zéolithes, l'aluminosillcate de magnésium, le sulfate de calcium, le carbonate de magnésium et/ou l'oxyde de magnésium.

9. Utilisation selon l'une quelconque des revendications 1 à 8,
**caractérisée en ce qu'**
on met en oeuvre en supplément du glycérol, du sorbitol et/ou des polyéthylèneglycols en tant qu'agent qui retient l'humidité.

10. Produits de soins buccaux et dentaires contenant :
de 15 à 25 % en poids de produit abrasif et de polissage,
de 30 à 65 % en poids de produit qui conserve l'humidité
de 1 à 10 % en poids de sulfate d'ester de polyglycol et d'acide gras, le cas échéant en mélange avec des co-agents tensioactifs,
de 1 à 2 % en poids de substances odoriférantes, et
de 0 à 5 % en poids d'autres adjuvants
avec la précision que les quantités utilisées se complètent à 100 % en poids.
